# EUROPÄISCHE PATENTSCHRIFT

(11) **EP 0 628 547 B1**
(45) Veröffentlichungstag und Bekanntmachung des Hinweises auf die Patenterteilung: **24.09.1997**
(21) Anmeldenummer: 94108435.2
(22) Anmeldetag: 01.06.1994
(51) Int. Cl.: C07D 213/89

(54) **Verfahren zur Herstellung von N-Aminopyridonen**
Process for the preparation of N-aminopyridones
Procédé pour la préparation des N-aminopyridones

(30) Priorität: 14.06.1993 DE 4319675
(43) Veröffentlichungstag der Anmeldung: 14.12.1994
(73) Patentinhaber: BASF Aktiengesellschaft, 67063 Ludwigshafen (DE)
(72) Erfinder: Beckmann, Stefan, Dr., D-68167 Mannheim (DE); Schefczik, Ernst, Dr., D-67069 Ludwigshafen (DE)

(56) Entgegenhaltungen:
- WO-A-92/19684
- DE-A- 2 150 772
- POLISH JOURNAL OF CHEMISTRY, Bd.58, 1984, WARSZAWA, PL Seiten 85 - 95 R. BALICKI 'Studies in the field ...'
- CHEMICAL ABSTRACTS, vol. 114, no. 8, 25. Februar 1991, Columbus, Ohio, US; abstract no. 64264u, Seite 113 ;
- CHEMICAL ABSTRACTS, vol. 92, no. 25, 23. Juni 1980, Columbus, Ohio, US; abstract no. 215333w, Seite 616 ;
- CHEMICAL ABSTRACTS, vol. 83, no. 17, 27. Oktober 1975, Columbus, Ohio, US; abstract no. 147421k, Seite 494 ;

## Beschreibung

Die vorliegende Erfindung betrifft ein neues Verfahren zur Herstellung von l-Benzoylamino-3-cyano-4-methyl-6- hydroxypyrid-2-on durch Umsetzung von acylierten Essigsäurehydrazinen mit Derivaten von Essigsäureestern.

Aus Polish J. Chem., Band 58, Seiten 85 bis 95, 1984, ist die Herstellung von N-aminosubstituierten 6-Hydroxypyrid-2-onen, die keine Substituenten an der Aminogruppe aufweisen, durch Umsetzung von acylierten Essigsäureestern mit Cyanacethydrazid bekannt.

In der DE-A-2 150 772 ist die Herstellung eines 1-Acetylaminopyridons beschrieben.

Aufgabe der vorliegenden Erfindung war es, ein neues Verfahren zur Herstellung von l-Benzoylamino-3-cyano-4-methyl-6- hydroxypyrid-2-on bereitzustellen, dabei sollte die Benzoylgruppe schon vor der Pyridonbildung vorhanden sein, d.h. nicht nachträglich in den fertigen Pyridonring eingeführt werden.

Es wurde nun gefunden, daß die Herstellung des Pyridons der Formel I 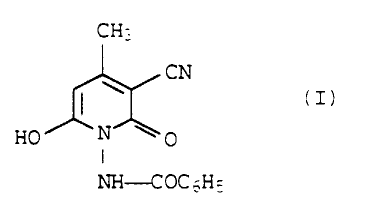 vorteilhaft gelingt, wenn man
a) in einem 1. Schritt Cyanessigsäure-C₁-C₄-alkylester mit Hydrazin in Wasser als Reaktionsmedium umsetzt, anschließend das resultierende Cyanessigsäurehydrazid ohne Zwischenisolierung
b) in einem 2. Schritt in Gegenwart eines Puffersystems mit Benzoylchlorid behandelt und das resultierende N-Benzoylcyanessigsäurehydrazid danach ohne Zwischenisolierung
c) in einem 3. Schritt in Gegenwart einer Base und eines Phasentransferkatalysators mit Acetessigsäure-C₁-C₄-alkylester umsetzt.

Die obengenannte Formel I und die obengenannten Verbindungen umfassen selbstverständlich auch die entsprechenden tautomeren Formen der einzelnen Verbindungen. Beispielhaft seien dafür aufgeführt: 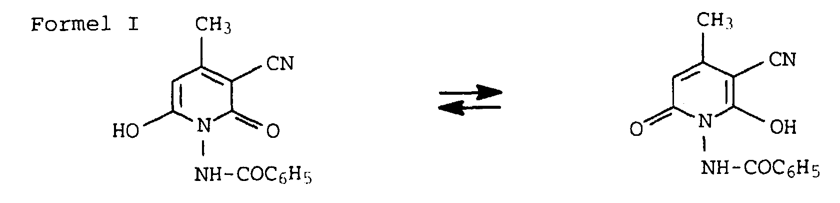 Acetessigsäureester 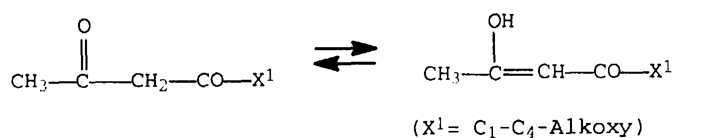 N-Benzoylcyanessigsäurehydrazid

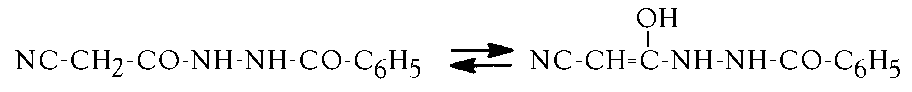

Geeignete Basen, die im erfindungsgemäßen Verfahren zur Anwendung kommen, sind z.B. Alkalihydroxide, wie Lithium-, Natrium- oder Kaliumhydroxid, Alkalicarbonate, wie Lithium-, Natrium- oder Kaliumcarbonat, oder Alkalialkanolate, wie Lithium-, Natrium- oder Kaliummethanolat, -ethanolat oder -propanolat.

Geeignete Cyanessigsäure-C₁-C₄-alkylester oder Acetessigsäure-C₁-C₄-alkylester sind z.B. die Methyl-, Ethyl-, Propyl-, Isopropyl-, Butyl- oder Isobutylester. Die Anwendung der jeweiligen Methyl- oder Ethylester ist bevorzugt.

Der 1. Schritt wird im allgemeinen bei einer Temperatur von 0 bis 50°C, vorzugsweise 20 bis 30°C, durchgeführt.

Cyanessigsäure-C₁-C₄-alkylester und Hydrazin kommen dabei in der Regel im Molverhältnis 1:1 bis 1:3, vorzugsweise 1:1 bis 1:2 und insbesondere 1:1 bis 1:1,1 zur Anwendung.

Bezogen auf das Gewicht von Cyanessigsäure-C₁-C₄-alkylester wendet man üblicherweise 100 bis 300 Gew.-%, vorzugsweise 200 bis 250 Gew.-%, Wasser als Reaktionsmedium an.

Nach Bildung des Cyanessigsäurehydrazids, was im allgemeinen ca. 8 Stunden in Anspruch nimmt, erfolgt ohne Zwischenisolierung des Hydrazids die Behandlung mit Benzoylchlorid. Diese findet in der Regel bei einer Temperatur von 0 bis 50°C, vorzugsweise 20 bis 30°C, und in Gegenwart eines Puffersystems, z.B. eines Phosphatpuffers auf Basis von Dikaliumhydrogenphosphat, statt.

Je mol Cyanessigsäure-C₁-C₄-alkylester (in Schritt 1) wendet man üblicherweise 1 bis 1,5 mol, vorzugsweise 1 bis 1,1 mol, Benzoylchlorid an.

Nach Bildung des N-Benzoylcyanessigsäurehydrazids, was im allgemeinen 5 bis 10 Stunden beansprucht, erfolgt ohne Zwischenisolierung des N-Benzoylcyanessigsäurehydrazids die Umsetzung mit Acetessigsäure-C₁-C₄-alkylester. Diese findet in der Regel bei einer Temperatur von 0 bis 50°C, vorzugsweise 20 bis 30°C, in Gegenwart eines Phasentransferkatalysators, z.B. Trimethylbenzylammoniumchlorid oder Tetrabutylammoniumchlorid, statt.

Man arbeitet dabei weiterhin in Gegenwart einer Base, wobei man vorteilhaft einen pH-Wert des Reaktionsgemisches von 9,5 bis 10 einhält. Geeignete Basen sind z.B. die oben genannten. Die Verwendung von 20 bis 50 gew.-%iger Natronlauge oder Kalilauge ist bevorzugt.

Je mol Cyanessigsäure-C₁-C₄-alkylester (in Schritt 1) wendet man in der Regel 1 bis 3 mol, vorzugsweise 1 bis 2 mol, Acetessigsäure-C₁-C₄-alkylester an.

Der Phasentransferkatalysator kommt in katalytischen Mengen, das sind z.B. 0,01 bis 0,2 mol, bezogen auf 1 mol Cyanessigsäure-C₁-C₄-alkylester (in Schritt 1), zur Anwendung.

Nach einer Reaktionsdauer von 10 bis 20 Stunden ist die Umsetzung beendet und das resultierende Pyridon der Formel I wird nach Ansäuern des Reaktionsgemisches, z.B. mit konz. Salzsäure, abgetrennt, gewaschen und getrocknet.

Mittels der neuen Verfahren wird das Zielprodukt auf einfache Weise und in hoher Ausbeute und Reinheit erhalten. Dies war nicht vorauszusehen, da bei der Umsetzung der multifunktionellen Reaktionspartner verstärkt mit der Bildung von Nebenprodukten (Konkurrenzprodukten) zu rechnen war.

Das Pyridon der Formel I ist ein wertvolles Zwischenprodukt für die Herstellung von Pyridonfarbstoffen, wie sie beispielsweise in der WO-A-92/19684 beschrieben sind.

Das folgende Beispiel soll die Erfindung näher erläutern.

### Beispiel

5 g Hydrazinhydrat wurden unter Eiskühlung zu einem Gemisch von 10 ml Wasser und 9,9 ml Cyanessigsäuremethylester getropft. Es wurde dann 5 Stunden bei Raumtemperatur nachgerührt. Anschließend wurden 8,7 g Dikaliumhydrogenphosphat, gelöst in 40 ml Wasser, zum Reaktionsgemisch gegeben. Dieses wurde dann auf -5 bis 0°C abgekühlt und tropfenweise mit 14,05 g Benzoylchlorid versetzt. Danach wurde das Gemisch auf Raumtemperatur erwärmt und mit 13,5 ml 25 gew.-%iger Natronlauge versetzt, so daß sich ein pH-Wert von 9,9 einstellte. Es wurden 1,9 g Trimethylbenzylammoniumchlorid und 10,8 ml Acetessigsäuremethylester zugegeben, wobei ein pH-Wert von 9,3 resultierte. Das Reaktionsgemisch wurde 20 min bei Raumtemperatur gerührt, danach wurden 24 ml 25 gew.-%ige Natronlauge zugegeben, wobei ein pH-Wert von 10 resultierte, und 5 Stunden bei Raumtemperatur nachgerührt. Danach wurden weitere 7,6 ml Acetessigsäuremethylester und weitere 2 ml 25 gew.-%ige Natronlauge hinzugegeben. Nach 3,5-stündigem Nachrühren wurde das Reaktionsgemisch mit 25 ml konz. Salzsäure versetzt und der resultierende Niederschlag abgesaugt, mit Wasser gewaschen und getrocknet. Man erhielt 20,2 g des Pyridons der Formel 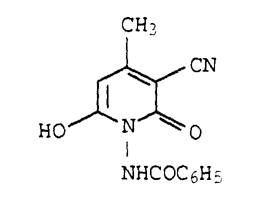 (Schmelzpunkt: 247°C)

## Patentansprüche

1. Verfahren zur Herstellung des Pyridons der Formel I 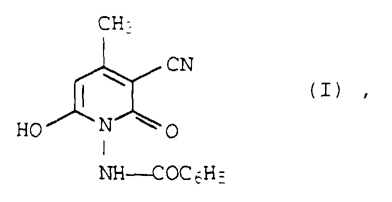 dadurch gekennzeichnet, daß man
a) in einem 1. Schritt Cyanessigsäure-C₁-C₄-alkylester mit Hydrazin in Wasser als Reaktionsmedium umsetzt, anschließend das resultierende Cyanessigsäurehydrazid ohne Zwischenisolierung
b) in einem 2. Schritt in Gegenwart eines Puffersystems mit Benzoylchlorid behandelt und das resultierende N-Benzoylcyanessigsäurehydrazid danach ohne Zwischenisolierung
c) in einem 3. Schritt in Gegenwart einer Base und eines Phasentransferkatalysators mit Acetessigsäure-C₁-C₄-alkylester umsetzt.

2. Verfahren nach Anspruch 1, dadurch gekennzeichnet, daß man die Schritte 1 bis 3 jeweils bei einer Temperatur von 0 bis 50°C durchführt.

3. Verfahren nach Anspruch 1, dadurch gekennzeichnet, daß man im 1. Schritt Cyanessigsäure-C₁-C₄-alkylester und Hydrazin im Molverhältnis 1 : 1 bis 1 : 3 umsetzt.

4. Verfahren nach Anspruch 1, dadurch gekennzeichnet, daß man im 2. Schritt 1 bis 1,5 mol Benzoylchlorid, bezogen auf 1 mol Cyanessigsäure-C₁-C₄-alkylester (1. Schritt), anwendet.

5. Verfahren nach Anspruch 1, dadurch gekennzeichnet, daß man im 3. Schritt 1 bis 3 mol Acetessigsäure-C₁-C₄-alkylester, bezogen auf 1 mol Cyanessigsäure-C₁-C₄-alkylester (1. Schritt), anwendet.

## Claims

1. A process for preparing the pyridone of the formula I 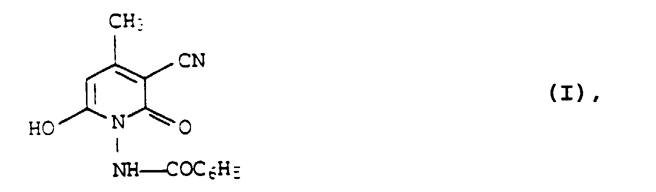 which comprises
a) in a first step, reacting a C₁-C₄-alkyl cyanoacetate with hydrazine in water as reaction medium to form a cyanoacetic hydrazide, then
b) in a second step, treating the as-synthesized cyanoacetic hydrazide with benzoyl chloride in the presence of a buffer system to form an B-benzoylcyanoacetic hydrazide, and thereafter
c) in a third step, reacting the as-synthesized N-benzoylcyanoacetic hydrazide with a C₁-C₄-alkyl acetoacetate in the presence of both a base and a phase transfer catalyst.

2. A process as claimed in claim 1, wherein steps 1 to 3 are each carried out at from 0 to 50°C.

3. A process as claimed in claim 1, wherein, in step 1, the C₁-C₄-alkyl cyanoacetate and hydrazine are reacted in a molar ratio of from 1:1 to 1:3.

4. A process as claimed in claim 1, wherein, in step 2, from 1 to 1.5 mol of benzoyl chloride are used, based on 1 mol of C₁-C₄-alkyl cyanoacetate in step 1.

5. A process as claimed in claim 1, wherein, in step 3, 1 to 3 mol of C₁-C₄-alkyl acetoacetate are used, based on 1 mol of C₁-C₄-alkyl cyanoacetate in step 1.

## Revendications

1. Procédé de préparation de la pyridone de formule I 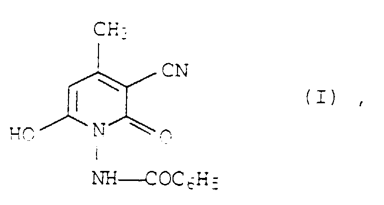 caractérisé en ce que
a) dans une première étape, on fait réagir un ester alkylique en C₁-C₄ de l'acide cyanacétique avec de l'hydrazine dans l'eau comme milieu réactionnel, puis on traite l'hydrazide d'acide cyanacétique obtenu, sans l'isoler,
b) dans une deuxième étape, avec du chlorure de benzoyle, en présence d'un tampon, et on fait ensuite réagir le N-benzoylhydrazide d'acide cyanacétique obtenu, sans l'isoler,
c) dans une troisième étape, avec un ester alkylique en C₁-C₄ de l'acide acétoacétique, en présence d'une base et d'un catalyseur de transfert de phases.

2. Procédé selon la revendication 1, caractérisé en ce que l'on effectue chacune des étapes 1 à 3 à une température de 0 à 50°C.

3. Procédé selon la revendication 1, caractérisé en ce que l'on fait réagir, dans la première étape, un ester alkylique en C₁-C₄ de l'acide cyanacétique et de l'hydrazine, dans un rapport en moles de 1:1 à 1:3.

4. Procédé selon la revendication 1, caractérisé en ce que l'on utilise, dans le deuxième étape, 1 à 1,5 moles de chlorure de benzoyle, pour 1 mole d'ester alkylique en C₁-C₄ de l'acide cyanacétique (première étape).

5. Procédé selon la revendication 1, caractérisé en ce que l'on utilise, dans la troisième étape, 1 à 3 moles d'ester alkylique en C₁-C₄ de l'acide acétoacétique, pour 1 mole d'ester alkylique en C₁-C₄ de l'acide cyanacétique (première étape).
